# EUROPÄISCHE PATENTSCHRIFT

(11) **EP 1 581 190 B1**
(45) Veröffentlichungstag und Bekanntmachung des Hinweises auf die Patenterteilung: **23.01.2013**
(21) Anmeldenummer: 03785797.6
(22) Anmeldetag: 11.12.2003
(51) Int. Cl.: A61K 9/16

(54) **GRANULAT MIT ÖLIGER SUBSTANZ, HERSTELLUNGSVERFAHREN UND TABLETTE**
GRANULATE COMPRISING AN OILY SUBSTANCE, CORRESPONDING PRODUCTION METHOD AND TABLET
GRANULAT DOTE D'UNE SUBSTANCE HUILEUSE, PROCEDE DE REALISATION ASSOCIE ET COMPRIME

(30) Priorität: 09.01.2003 DE 10300325
(43) Veröffentlichungstag der Anmeldung: 05.10.2005
(73) Patentinhaber: Hexal AG, 83607 Holzkirchen (DE)
(72) Erfinder: KLOKKERS, Karin, 83620 Vagen (DE); OTTO, Ina, Elfriede, 82024 Taufkirchen (DE); MEYER, Heidemarie, Edith, Elfriede, 39108 Magdeburg (DE)
(74) Vertreter: Boeters, Hans Dietrich
(86) Internationale Anmeldenummer: PCT/EP2003/014097
(87) Internationale Veröffentlichungsnummer: WO 2004/062644

(56) Entgegenhaltungen:
- EP-A- 0 255 002
- EP-A- 0 594 152
- WO-A-99/01111
- US-A- 4 128 658
- US-A- 5 472 704
- DATABASE WPI Section Ch, Week 199724 Derwent Publications Ltd., London, GB; Class A11, AN 1997-267703 XP002277155 & JP 09 095439 A (EISAI CO LTD) 8. April 1997 (1997-04-08)
- DATABASE WPI Section Ch, Week 199713 Derwent Publications Ltd., London, GB; Class A11, AN 1997-140843 XP002277156 & JP 09 020686 A (TEIKOKU SEIYAKU KK) 21. Januar 1997 (1997-01-21)

## Beschreibung

Die Erfindung bezieht sich auf Retard-Formulierungen für korrosive und/oder hydrophile Wirkstoffe. Die Herstellung dieser Retard-Formulierungen erfolgt erfindungsgemäß mit Hilfe eines Granulators, in dem eine Mischung aus Wirkstoff(en) und Retardierungsmittel(n) mit einer öligen Substanz besprüht wird.

Eine Möglichkeit Retard-Formulierungen zu erhalten, besteht üblicherweise in der Herstellung von Tabletten oder Kapseln, die den pharmazeutischen Wirkstoff dispergiert in einer Matrix enthalten. Die Matrix bildet eine kontinuierliche Phase um den Wirkstoff und erlaubt so eine graduelle Freisetzung. Ein Vorteil einer Retard-Formulierung ist das gleichmäßige und langanhaltende effektive Wirkstoffniveau bei Freisetzung. Der zeitliche Abstand zwischen den einzelnen Tabletteneinnahmen ist für retardierte Arzneiformen größer als bei schnellfreisetzenden Formulierungen. So kann eine bessere Patienten-Compliance erreicht werden.

Bekannte Verfahren zur Herstellung von Retard-Formulierungen mit lipophiler Matrix sind insbesondere u.a. die Schmelzgranulierung sowie die Schmelzextrusion.

EP 0 630 235 B1 offenbart den Prozeß der Schmelzpelletierung. In einem Pflugscharmischer wird der Wirkstoff mit wasserunlöslichen, wachsartigen Bindemitteln (Smp. > 40 °C) zu Pellets verarbeitet. Gemäß EP 0 654 263 A1 wird so nur eine geringe Ausbeute an Pellets im gewünschten Größenbereich erzielt.

In EP 0 654 263 A1 wird ein verbessertes Verfahren zur Herstellung von sustained-release Partikeln durch Schmelzgranulierung beschrieben. In einem Hochgeschwindigkeitsmischer werden Wirkstoff-Teilchen mit einem schmelzbaren Träger (z. B. Öle, Wachse, Smp. 35-150 °C) und optional mit einer freisetzungskontrollierenden Komponente (Salze, Lactose, HPMC) gemischt, wobei das Trägermaterial durch Energiezufuhr erweicht wird. Die so erhaltenen, abgekühlten Agglomerate werden auf einen Durchmesser < 2 mm gebracht. Die Maschenweite der Siebe darf nicht zu klein sein, sonst werden sie durch die unter Druck schmelzenden Agglomerate verstopft. Die gesiebten Partikel werden erneut im Hochgeschwindigkeitsmischer erweicht, so daß die feinen Partikel von den größeren aufgenommen werden. Ein geringer Prozentsatz des Trägers kann erneut zugesetzt werden. So wird eine gute Ausbeute an Partikeln bestimmter Größe und gleichmäßiger Freisetzungsrate, insbesondere für sehr gut wasserlösliche Substanzen, erhalten. Allerdings verringert sich die Freisetzungsrate nach dem Verpressen der Partikel zu Tabletten.

Gemäß EP 0 789 559 B2 kann der Prozeß von EP 0 654 263 A1 hinsichtlich Ausbeute, Wirkstoffbeladung und Einheitlichkeit der Partikelgröße verbessert werden, wenn die Agglomerate aus Wirkstoff und schmelzbarem Träger extrudiert werden.

EP 0 731 694 B1, EP 1 023 896 A2, EP 624 366 B1 und EP 729 751 A1 beschreiben Matrixformulierungen für Tramadol mit hydrophilen/hydrophoben Polymeren, substituierten/unsubstituierten C8-C50 Kohlenwasserstoffen (Fettsäuren, Pflanzenöle, Wachse) oder Polyalkylenglycolen als Retardierungsmittel. Die sustained-release Matrizes können beispielsweise durch Naßgranulierung des Wirkstoffs mit Cellulosederivaten, Vermischen der Granulate mit Fettalkohol und anschließendes Pressen und Formen der Granulate hergestellt werden. Auch eine Beschichtungstechnik ist möglich. Der Wirkstoff kann auch mit einem Bindemittel, das einen Smp. über 40 °C aufweist (z.B. hydrierte Pflanzenöle), beispielsweise in einem Pflugschar-Mischer pelletiert werden.

US 4,013,784 betrifft Retard-Formulierungen mit einer Fettmatrix aus Triglyceriden mit C12-C18 Fettsäuren. Mit Hilfe eines Hochgeschwindigkeitsmischers wird der Wirkstoff mit Calciumsalzen in einem (oder mehreren) geschmolzenen Triglycerid(en) dispergiert. Die Granulate lassen sich aus der Dispersion durch einen Hochdruck-Atomizer, eine vibrierende Düse oder durch Erkaltenlassen in Platten mit anschließendem Zerschneiden herstellen.

In US 4,132,753 wird die Herstellung von sustained-release Granulaten durch Infrarot-Bestrahlung einer Mischung aus pulverförmigem Wirkstoff und feinteiligem, wachsartigem Material in einem "rotating tumling cylinder" beschrieben. Hier wird der Wirkstoff über den Schmelzpunkt des wachsartigen Materials erhitzt und sinkt in das noch nicht geschmolzene wachsartige Material ein.

In WO 92/06679 wird ein verbessertes Verfahren der Schmelzgranulierung offenbart, indem der Wirkstoff in kohäsiver Form (Partikelgröße < 20µm) und z. B. ein lipophiles Bindemittel eingesetzt werden. So können Pellets mit geringer Porösität erzielt werden.

WO 93/07859 und WO 96/14058 beschreiben die Herstellung von sustained-release Matrix-Formulierungen mit Hilfe der Schmelzextrusionstechnologie.

EP 0 043 254 A1 betrifft die Verwendung eines niedrigschmelzenden und eines hochschmelzenden Lipids zur Herstellung einer Matrix-Retard-Formulierung mit Hilfe von Extrusions- oder Granulierungstechnik. Ziel ist es, den Wirkstoff thermisch wenig zu belasten.

WO 99/01111 offenbart eine sustained-release Tramadol-Formulierung mit einem stabilen Freisetzungsprofil, das durch "curing" der festen, wachsartige Substanzen enthaltenden Matrix-Formulierung für einen bestimmten Zeitraum bei einer bestimmten Temperatur erzielt wird. Die Herstellung erfolgt bevorzugt über Extrusion und Pelletierung.

WO 98/52684 beschreibt ein verbessertes Verfahren der Schmelzextrusion mit einer integrierten, speziellen Kühlzone im Schneckenextruder.

In EP 0 914 823 A1 wird eine Tramadol-Retard-Formulierung mit einem Fettalkohol als Matrixbildner beschrieben. Mit dem Fettalkohol wurde ein Matrixbildner gefunden, der eine Freisetzung des Wirkstoffs über 24 Std. ermöglicht. Hier wird der Wirkstoff in einem Wirbelschichtgranulator mit mikrokristalliner Cellulose gemischt und mit einer isopropanolischen Lösung des Fettalkohols besprüht. Nach dem Sieben erhält man ein gut fließfähiges Granulat, das zu Tabletten verpresst wird.

In WO 99/65471 wird ein Verfahren zur Herstellung von Retard-Tabletten beschrieben, bei dem eine flüssige Öl-in-Wasser-Emulsion auf eine wirkstoffhaltige Pulvermischung oder ein Granulat gesprüht wird. Das so erhaltene fettüberzogene Granulat wird zu Tabletten gepresst. Nur die Tabletten, aber nicht die Granulate zeigen eine verzögerte Wirkstoff-Freisetzung.

In EP 0 665 830 B1 wird das Problem der Korrosivität von Tilidin-Hydrochlorid-Semihydrat durch die Verwendung von Tilidindihydrogenorthophosphat umgangen. Das Phosphat-Salz ist im Gegensatz zum Hydrochlorid-Salz in keiner Weise hygroskopisch und reagiert daher nicht mit metallischen Werkstoffen unter Korrosion.

Vorzüge der Schmelzextrusion sind a) eine hohe Wirkstoffbeladung, auch für wasserlösliche Wirkstoffe, b) eine hohe Dichtigkeit und c) eine geringe Porösität der Pellet- bzw. Partikeloberfläche und damit verbunden eine gute Retardierung. Die Extrusion ist daher eine oft gewählte Methode zur Herstellung von Retard-Formulierungen.

Nachteilig bei den Extrusionsverfahren ist, daß beim Abkühlen der geschmolzenen Massen -aufgrund der unterschiedlichen physikalischen Eigenschaften der Komponenten - zum einen eine Entmischung auftreten kann, zum anderen aber auch bei Verwendung oligomerer oder polymerer Substanzen ein Molekulargewichtsabbau erfolgen kann. Letzteres kann zu einer eingeschränkten Wirkung der retardierenden Polymere führen. Auch die Produkthomogenität ist häufig unbefriedigend.

Die Schmelzextrusion ist ein kontinuierliches Verfahren mit hohem Energie-Einsatz und daher zeit- und kostenintensiv.

Bei der Schmelzeinbettung in einem beheizbaren Mischer als auch beim Extrusionsverfahren sind die große Anzahl der Verfahrensschritte sowie die Staubproblematik an den Schnittstellen der verschiedenen Verfahrensschritte ein Nachteil. In der Regel sind die so gewonnen Einbettungen oder Extrudate zu grob für eine direkte Weiterverarbeitung. Sie müssen unter aufwendigen Bedingungen gesiebt werden, d.h. die Produktion ist zeit- und kostenintensiv. Zudem kommt es bei diesen Verfahren zu einer unerwünschten Wärmebelastung der wirkstoffhaltigen Mischungen.

Bei der Wirbelschichtgranulierung von Wirkstoffen mit Fetten oder Wachsen erhält man nach den bisher bekannten Verfahren wenig kompakte und sehr poröse Granulatkörner, die keine ausreichende Retardierung der Wirkstofffreisetzung aufweisen.

Besondere Probleme ergeben sich bei der Verarbeitung von korrosiven Wirkstoffen. Beispiel für einen korrosiven Wirkstoff ist Tilidin-Hydrochlorid, das stark hygroskopisch ist und mit metallischen Oberflächen (z. B. Tablettenstempel) reagiert. Bei der Herstellung von Tabletten oder Granulaten mit diesen Wirkstoffen sind daher besondere Anforderungen für die Klimatisierung der Arbeitsräume und an den Korrosionsschutz der verwendeten Apparaturen und Werkzeuge zu erfüllen.

Aufgabe der Erfindung ist ein einfacher und effektiver Prozeß zur Herstellung von Retard-Formulierungen mit Hilfe von Granulatoren, wobei die Formulierungen ein definiertes sustained-release Freisetzungsprofil für den(die) Wirkstoff(e) aufweisen sollen. Der Herstellprozeß soll allgemein für verschiedene Wirkstoffe und unterschiedliche Freisetzungsprofile anwendbar sein. Das Verfahren soll sich außerdem für korrosive und/oder hydrophile Wirkstoffe eignen.

Gemäß einer Ausführungsform wird die der Erfindung zugrundeliegende Aufgabe durch ein Verfahren zur Herstellung eines Granulats für eine Arzneimittelformulierung gelöst, bei dem man
(i) eine Mischung umfassend oder bestehend aus
   - einem oder mehreren Wirkstoffen und
   - einem oder mehreren Retardierungsmitteln mit einer öligen Substanz benetzt und
(ii) die Mischung granuliert.

Gemäß einer weiteren Ausführungsform wird die der Erfindung zugrundeliegende Aufgabe durch ein Verfahren zur Herstellung eines Granulats für eine Arzneimittelformulierung gelöst, bei dem man
(i) einen oder mehrere Wirkstoffe mit einem oder mehreren Retardierungsmitteln mischt,
(ii) die erhaltene Mischung mit einer öligen Substanz benetzt und
(iii) die erhaltene Mischung granuliert.

Bei dem erfindungsgemäßen Verfahren kann man eine Mischung (i) gemäß der ersten Ausführungsform oder eine Mischung (ii) gemäß der zweiten Ausführungsform mit einem Gehalt an einem oder mehreren Hilfsstoffen verwenden, insbesondere mit einem Gehalt an einem oder mehreren Füllmitteln, Fließregulierungsmitteln, Netzmitteln und/oder Sprengmitteln.

Ferner kann man bei dem erfindungsgemäßen Verfahren mit der öligen Substanz durch Aufsprühen benetzen.

Ferner kann man bei dem erfindungsgemäßen Verfahren bei Raumtemperatur mit der öligen Substanz benetzen.

Ferner kann man bei dem erfindungsgemäßen Verfahren für die Mischung (i) gemäß der ersten Ausführungsform oder für die Mischung (ii) gemäß der zweiten Ausführungsform mindestens einen korrosiven und/oder hydrophilen Wirkstoff vorsehen. Ein Beispiel für den Stand der Technik korrosiver Wirkstoffe liefert EP 0 665 830.

Ferner kann man bei dem erfindungsgemäßen Verfahren einen Wirkstoffgehalt von 0,1 bis 98 und insbesondere 0,5 bis 70 Gew.-% vorsehen (auf Basis Granulat-Gesamtgewicht).

Ferner kann man bei dem erfindungsgemäßen Verfahren für die Mischung (i) gemäß der ersten Ausführungsform oder für die Mischung (ii) gemäß der zweiten Ausführungsform als Retardierungsmittel ein lipophiles Retardierungsmittel bzw. einen Fettmatrix-Bildner vorsehen, insbesondere in Kombination mit einem Hydrogelmatrix-Bildner und/oder Gerüstmatrix-Bildner.

Ferner kann man bei dem erfindungsgemäßen Verfahren als Retardierungsmittel eine Kombination aus Fettmatrix-Bildner und Hydrogelmatrix-Bildner vorsehen.

Ferner kann man bei dem erfindungsgemäßen Verfahren als Retardierungsmittel eine Kombination aus Fettmatrix-Bildner und Gerüstmatrix-Bildner mit wasserlöslichem Hilfsstoff vorsehen.

Ferner kann man bei dem erfindungsgemäßen Verfahren als ölige Substanz ein natürliches Öl, ein synthetisches Öl, eine Lösung von Wachs in Öl oder dünnflüssiges Wachs verwenden.

Ferner kann man bei dem erfindungsgemäßen Verfahren einen Gehalt an öliger Substanz von 0,2 bis 20 und insbesondere 1 bis 7,5 Gew.-% vorsehen (auf Basis Granulat-Gesamtgewicht).

Ferner kann man bei dem erfindungsgemäßen Verfahren das erhaltene Granulat zusätzlich mit einer Aussenphase aus einem oder aus mehreren Retardierungsmitteln versehen.

Ferner kann man bei dem erfindungsgemäßen Verfahren mit einem Wirbelschichtgranulator oder einem Pflugscharmischer granulieren.

Ferner kann man bei dem erfindungsgemäßen Verfahren mit Hilfe eines Granulatbindemittels granulieren, insbesondere in Form einer Lösung (Granulierlösung) des Granulatbindemittels in einem Lösungsmittel.

Schließlich kann man bei dem erfindungsgemäßen Verfahren das erhaltene Granulat zu Tabletten weiterverarbeiten.

Gemäß einer weiteren Ausführungsform betrifft die Erfindung ein Verfahren zur Herstellung von Tabletten, bei dem man ein Granulat zu Tabletten verarbeitet, das gemäß einem erfindungsgemäßen Verfahren zur Herstellung eines Granulats für eine Arzneimittelformulierung erhalten worden ist.

Bei der Weiterverarbeitung eines erfindungsgemäßen Granulats zu Tabletten oder bei der erfindungsgemäßen Herstellung von Tabletten kann man Hilfsstoffe verwenden, insbesondere Füllstoffe, Schmiermittel, Fließregulierungsmittel und/oder Sprengmittel.

Schließlich kann man eine erfindungsgemäß erhaltene Tablette mit einem Überzug versehen.

Gemäß einer weiteren Ausführungsform betrifft die Erfindung ein Granulat, das gemäß einem erfindungsgemäßen Verfahren erhalten worden ist.

Gemäß einer weiteren Ausführungsform betrifft die Erfindung ein Granulat für eine Arzneimittelformulierung, wobei das Granulat aus einer Mischung aus
- einem oder mehreren Wirkstoffen und
- einem oder mehreren Retardierungsmitteln besteht oder sie umfasst, wobei
- die Mischung mit einer öligen Substanz benetzt ist.

Das erfindungsgemäße Granulat kann mindestens einen korrosiven und/oder hydrophilen Wirkstoff enthalten oder umfassen.

Schließlich betrifft die Erfindung eine Tablette, die nach einem erfindungsgemäßen Verfahren erhalten worden ist.

Überraschenderweise wurde nun gefunden, daß eine hinreichende Retardierung und eine gute Verarbeitbarkeit eines Wirkstoffs auch mittels Granulierverfahren erreicht werden kann, wenn man eine Mischung aus Wirkstoff(en) und lipophilen Retardierungsmittel(n) (= Fettmatrix) vor oder während der Granulierung mit einer öligen Substanz besprüht. Die ölige Substanz benetzt bzw. befilmt die Oberfläche der Wirkstoffpartikel und hydrophobisiert diese. Die so erhaltenen, nicht-klebenden Granulate lassen sich gut weiterverarbeiten, z. B. sieben und tablettieren. Durch die Einbettung des Wirkstoffs in eine Fettmatrix in Kombination mit einer öligen Substanz wird ein direkter Kontakt des Wirkstoffs mit den Werkzeugoberflächen vermieden, d.h. es können auch korrosive Wirkstoffe zu Granulaten verarbeitet werden. Auch die Bildung von Verunreinigungen, die durch einen Kontakt des Wirkstoffs mit Metallionen der Werkzeugoberflächen katalysiert wird, läßt sich durch diese Art der Wirkstoffeinbettung stark verringern. Die Kombination aus Fettmatrix und öliger Substanz führt zu einer besonders guten Einbettung des/der Wirkstoffs/Wirkstoffe. Daraus resultiert eine gute Retardierung der Wirkstofffreisetzung.

Als Granulatoren können Wirbelschichtgeräte oder Pflugscharmischer eingesetzt werden. Bevorzugt wird in einem Wirbelschichtgerät granuliert.

Bei dem erfindungsgemäßen Verfahren zur Herstellung von Retard-Formulierungen wird/werden zunächst der (die) Wirkstoff(e) mit mindestens einem lipophilen Retardierungsmittel (=Fettmatixbildner) und ggf. ein oder mehreren Hilfsstoffen (z. B. Füllmittel, Fließregulierungsmittel, Netzmittel und/oder Sprengmittel) in einem Wirbelschichtgranulator gemischt. Diese Mischung wird bei Raumtemperatur mit einer öligen Substanz besprüht. Nachfolgend wird eine bindemittelhaltige Granulierlösung auf die Partikel aufgesprüht. Bei diesem Aufsprühen können Partikel, bestehend aus Wirkstoff(en), Retardierungsmittel(n), ggf. Hilfsstoffen und öliger Substanz, eine Temperatur von 30 bis 40 °C aufweisen. Das so erhaltene Granulat kann optional mit einer Außenphase aus einem oder mehreren Retardierungsmitteln versehen werden. Nach dem Trocknen und Sieben erhält man ein fließfähiges Granulat mit einheitlicher Korngrößenverteilung.

Als bevorzugte Beispiele für korrosive Wirkstoffe seien genannt: Tilidin-Hydrochlorid, Ranitidin-Hydrochlorid, Clindamycin-Hydrochlorid, Doxepin-Hydrochlorid, Citalopram-Hydrobromid, Amitriptylin, Cetirizin und Piroxicam. Die korrosiven Wirkstoffe können als pharmazeutisch unbedenklichen Salze, Hydrate, Solvate sowie in Form von Derivaten eingesetzt werden. Die korrosiven Wirkstoffe können auch in Kombination mit weiteren nichtkorrosiven Wirkstoffen eingesetzt werden. Bevorzugt wird eine Kombination von Tilidin-Hydrochlorid und Naloxon-Hydrochlorid.

Die Wirkstoffgehalte können je nach eingesetztem Wirkstoff und gewünschter Freisetzungsgeschwindigkeit in weiten Grenzen variieren. So kann die Wirkstoffkombination im Bereich von 0,1 bis 98 Gew.%, vorzugsweise von 0,5 bis 70 Gew.% bezogen auf das Gesamtgewicht des für eine Arzneimittelformulierung vorgesehenen Granulats liegen.

Zu den Retardierungsmitteln zählen Fettmatrix-, Hydrogelmatrix- und Gerüstmatrixbildner.

Als lipophile Retardierungsmittel (=Fettmatrixbildner) eignen sich beispielsweise
- Fettalkohole wie Stearylalkohol;
- Mono-, Di- und Triglyceride wie Glycerinmonostearat, Precirol (Glycerinpalmitostearat) oder Compritol (Glycerinmonobehenat mit 0.2-0.3% Magnesiumstearat);
- hydrierte Pflanzenöle wie hydriertes Rhizinusöl (Cutina HR);
- Wachse wie Bienen-, Carnauba- oder mikrokristallines Wachs.

Als bevorzugte lipophile Retardierungsmittel werden Glycerinmonobehenat und/oder hydriertes Rhizinusöl eingesetzt. Die Fettmatrixbildner sind in Konzentrationen von 5 bis 60 Gew.%, insbesondere von 10 bis 50 Gew.% bezogen auf das Gesamtgewicht der Arzneiform enthalten.

In Kombination mit einem oder mehreren Fettmatrixbildnern können auch Hydrogelmatrixbildner verwendet werden wie z. B. Hydroxypropylmethylcellulose, Hydroxypropylcellulose, Ethylhydroxyethylcellulose, Hydroxyethylcellulose, Methylcellulose, Alginate, Carbomer (Polyacrylsäuren), Natriumcarboxymethylcellulose, Tragant Gummi oder Gelatine. Diese Polymere können hydratisieren und eine gelartige Schicht bilden, welche langsam durch Diffusion und Erosion den Wirkstoff freisetzen kann.

Bei einer weiteren kontrolliert freisetzenden Matrixform wird/werden der/die Wirkstoff(e) zusammen mit wasserlöslichen Hilfsstoffen in ein Gerüst, gebildet aus wasserunlöslichen, unverdaulichen Hilfsstoffen eingebettet. Durch Herauslösen der löslichen Bestandteile entstehen Poren, durch die der Wirkstoff nach außen diffundieren kann. Als Gerüstmatrixbildner können Polymere wie z. B. Ethylcellulose, Celluloseacetat oder Polymethylmethacrylate eingesetzt werden.

Bevorzugt wird eine Kombination aus Fett- und Hydrogelmatrix verwendet.

Als ölige Substanz eignen sich Neutral-, Sesam-, Erdnuß-, Oliven-, Mandel-, Rhizinus-, Sojabohnen-, Kokos-, Baumwollsamen-, Mais-, Raps-, Sonnenblumen-, Weizenkeimöl sowie flüssiges Paraffin. Es können auch Wachslösungen in organischen Ölen oder dünnflüssiges Wachs verwendet werden. Besonders bevorzugt wird Neutralöl. Unter Neutralöl (Miglyol) versteht man eine Mischung von kurz- und mittelkettigen Triglyceride, vorwiegend mit den Fettsäuren Capryl- (C8) und Caprinsäure (C10). Zu den Miglyolen zählen auch Ester mit Propylenglykol. Bevorzugt wird Miglyol 812. Die öligen Substanzen sind in Konzentrationen von 0.2 bis 20 Gew.%, insbesondere von 1 bis 7,5 Gew.% bezogen auf das Gesamtgewicht des Granulats enthalten.

Folgende Hilfsstoffe können bei der Granulatherstellung eingesetzt werden: Fließregulierungsmittel wie z. B. Aerosil, Talkum; Granulatbindemittel wie z. B. Gelatine, Stärkekleister, Methylcellulose, Carboxymethylcellulose, Hydroxypropylmethylcellulose, Hydroxypropylcellulose, Pektin-Schleim, Polyvinylpyrrolidon, Polyvinylacetat und/oder Polyvinylalkohol; Trockenbindemittel wie z. B. mikrokristalline Cellulose, Stärke, modifizierte Stärke, Lactose und/oder Saccharose; Lösungsmittel für eine Granulierlösung wie z. B. Wasser, Ethanol, Isopropanol, Aceton oder deren Mischungen; Sprengmittel wie z. B. Natriumcarboxymethylstärke, Crospovidon; Netzmittel wie beispielsweise Natriumlaurylsulfat oder Natriumdocusat.

Die erfindungsgemäßen Granulate können zu Tabletten weiterverabeitet werden.

Für die Tablettenherstellung können folgende Hilfsstoffe verwendet werden:
- Füllstoffe wie Cellulose und/oder Cellulosederivate (z. B. mikrokristalline Cellulose), Zucker (z. B. Lactose, Glucose, Saccharose), Zuckeralkohole (z. B. Mannit, Sorbit), Stärke (z. B. Kartoffel-, Weizen-, Mais- und/oder Reisstärke),
- Schmiermittel wie Magnesiumstearat, Calciumstearat, Stearinsäure, hydrierte Pflanzenöle und/oder Talkum,
- Fließregulierungsmittel
- Sprengmittel

Die Tabletten können einen Überzug aufweisen.

Die Erfindung wird durch nachstehende Beispiele näher erläutert, ohne aber den Erfindungsumfang damit einzuschränken.

### Beispiel 1:

Die folgenden Stoffe werden zur Herstellung von Tilidin-Hydrochlorid Tabletten verwendet.

| **Bestandteile** | **Prozent (%)** | **Gewicht (mg/Tablette)** |
|---|---|---|
| Tilidin Hydrochlorid Hemihydrat | 25,7 | 102,87 |
| Naloxon Hydrochlorid | 2,3 | 8,80 |
| Hydroxypropylmethylcel lulose | 10,5 | 40,00 |
| Aerosil | 0,5 | 2,00 |
| Hydriertes Rhizinusöl | 17,9 | 68,50 |
| Compritol | 17,0 | 64,89 |
| Kollidon | 1,96 | 7,50 |
| Neutralöl | 5,0 | 19,11 |
| Gereinigtes Wasser | | 150,0 |
| Tablettose | 16,6 | 66,38 |
| Magnesiumstearat | 0,52 | 2,0 |
| Gesamt | 100 | 382,1 |

Tilidin Hydrochlorid Hemihydrat, Naloxon Hydrochlorid, Hydroxypropylmethylcellulose, Aerosil, Hydriertes Rhizinusöl und Compritol werden abgewogen, gesiebt und anschließend in einem Wirbelschichtgranulator gemischt. Diese Mischung wird im Wirbelschichtgranulator mit Neutralöl und dann mit einer Granulierlösung von Kollidon in Wasser besprüht. Das so erhaltene Granulat läßt man im Wirbelschichtgranulator trocknen. Nach dem Sieben durch ein 1 mm Sieb erhält man ein gut fließfähiges Granulat. Dieses wird in einem Freifallmischer mit Tablettose und Magnesiumstearat gemischt und zu Tabletten mit einem Gewicht von 382 mg je Tablette verpresst.

### Beispiel 2:

Die folgenden Stoffe werden zur Herstellung von Tabletten mit Tilidinmesilat verwendet.

| **Bestandteile** | **Prozent (%)** | **Gewicht (mg/Tablette)** |
|---|---|---|
| Tilidinmesilat | 29,8 | 119,25 |
| Naloxon Hydrochlorid | 2,2 | 8,80 |
| Microcellac | 17 | 67,95 |
| Hydroxypropylmethylcel lulose | 10 | 40,00 |
| Aerosil | 0,5 | 2,00 |
| Hydriertes Rhizinusöl | 17,1 | 68,50 |
| Compritol | 20 | 80,00 |
| Kollidon | 1,9 | 7,50 |
| Rhizinusöl | 1 | 4,00 |
| Gereinigtes Wasser | | 150,0 |
| Magnesiumstrearat | 0,5 | 2,00 |
| Gesamt | 100 | 400,0 |

Die Herstellung der Tabletten erfolgt analog zu Beispiel 1.

### Freisetzungsprofil

Apparatur zur Bestimmung der Tilidin-Freisetzung:
Zelle: Basket
Medium: 0.2 M Phosphatpuffer pH = 6,8
Temperatur: 37°C
Rührgeschwindigkeit: 150 UpM

| Zeit/min . | Freigesetztes Tilidin in % |
|---|---|
| 30 | 40 |
| 60 | 62 |
| 120 | 77 |
| 180 | 82 |
| 240 | 85 |
| 360 | 88 |
| 480 | 90 |
| 600 | 91 |
| 720 | 92 |

### Beispiel 3:

Die folgenden Stoffe werden zur Herstellung von Tabletten mit Tilidinmesilat verwendet.

| **Bestandteile** | **Prozent (%)** | **Gewicht (mg/Tablette)** |
|---|---|---|
| Tilidinmesilat | 31,2 | 119,25 |
| Naloxon Hydrochlorid | 2,3 | 8,8 |
| Hydroxypropylmethylcel lulose | 10,5 | 40,00 |
| Aerosil | 0,5 | 2,00 |
| Hydriertes Rhizinusöl | 17,9 | 68,50 |
| Compritol | 17,0 | 64,89 |
| Kollidon | 1,96 | 7,50 |
| Neutralöl | 5,0 | 19,11 |
| Gereinigtes Wasser | | 150,0 |
| Tablettose | 13,1 | 50,0 |
| Magnesiumstearat | 0,52 | 2,0 |
| Gesamt | 100 | 382,1 |

Die Herstellung der Tabletten erfolgt analog zu Beispiel 1.

### Freisetzungsprofil

Apparatur zur Bestimmung der Tilidin-Freisetzung:
Zelle: Basket
Medium: 0.2 M Phosphatpuffer pH = 6,8
Temperatur: 37°C
Rührgeschwindigkeit: 150 UpM

| Zeit/min . | Freigesetztes Tilidin in % |
|---|---|
| 30 | 29 |
| 60 | 46 |
| 120 | 62 |
| 180 | 69 |
| 240 | 74 |
| 360 | 81 |
| 480 | 85 |
| 600 | 88 |
| 720 | 90 |

## Patentansprüche

1. Verfahren zur Herstellung eines Granulats für eine Arzneimittelformulierung, bei dem man
(i) eine Mischung umfassend oder bestehend aus
- einem oder mehreren Wirkstoffen, und
- einem oder mehreren Retardierungsmitteln mit einer öligen Substanz, ausgewählt aus Neutralöl, Sesamöl, Erdnußöl, Olivenöl, Mandelöl, Sojabohnenöl, Kokosöl, Baumwollsamenöl, Maisöl, Rapsöl, Sonnenblumenöl, Weizenkeimöl, flüssigem Paraffin, Wachslösungen in organischen Ölen und dünnflüssigem Wachs, durch Aufsprühen benetzt und
(ii) die Mischung granuliert.

2. Verfahren zur Herstellung eines Granulats für eine Arzneimittelformulierung, bei dem man
(i) einen oder mehrere Wirkstoffe mit einem oder mehreren Retardierungsmitteln mischt,
(ii) die erhaltene Mischung mit einer öligen Substanz, ausgewählt aus Neutralöl, Sesamöl, Erdnußöl, Olivenöl, Mandelöl, Sojabohnenöl, Kokosöl, Baumwollsamenöl, Maisöl, Rapsöl, Sonnenblumenöl, Weizenkeimöl, flüssigem Paraffin, Wachslösungen in organischen Ölen und dünnflüssigem Wachs, durch Aufsprühen benetzt und
(iii)die erhaltene Mischung granuliert.

3. Verfahren nach Anspruch 1 oder 2, bei dem man eine Mischung gemäß Anspruch 1 (i) oder Anspruch 2 (ii) mit einem Gehalt an einem oder mehreren Hilfsstoffen verwendet, insbesondere mit einem Gehalt an einem oder mehreren Füllmitteln, Fließregulierungsmitteln, Netzmitteln und/oder Sprengmitteln.

4. Verfahren nach einem der vorhergehenden Ansprüche, bei dem man bei Raumtemperatur mit der öligen Substanz benetzt.

5. Verfahren nach einem der vorhergehenden Ansprüche, bei dem man für die Mischung gemäß Anspruch 1 (i) oder Anspruch 2 (ii) mindestens einen korrosiven und/oder hydrophilen Wirkstoff vorsieht.

6. Verfahren nach einem der vorhergehenden Ansprüche, bei dem man einen Wirkstoffgehalt von 0,1 bis 98 und insbesondere 0,5 bis 70 Gew.-% vorsieht (auf Basis Granulat-Gesamtgewicht).

7. Verfahren nach einem der vorhergehenden Ansprüche, bei dem man für die Mischung gemäß Anspruch 1 (i) oder Anspruch 2 (ii) als Retardierungsmittel ein lipophiles Retardierungsmittel vorsieht, insbesondere in Kombination mit einem Hydrogelmatrix- und/oder Gerüstmatrix-Bildner.

8. Verfahren nach Anspruch 7, bei dem man als Retardierungsmittel eine Kombination aus lipophilem Retardierungsmittel und Hydrogelmatrix-Bildner vorsieht.

9. Verfahren nach einem der vorhergehenden Ansprüche, bei dem man als Retardierungsmittel eine Kombination aus lipophilem Retardierungsmittel und Gerüstmatrix-Bildner mit wasserlöslichem Hilfsstoff vorsieht.

10. Verfahren nach einem der vorhergehenden Ansprüche, bei dem man als ölige Substanz ein natürliches Öl, ein synthetisches Öl, eine Lösung von Wachs in Öl oder dünnflüssiges Wachs verwendet.

11. Verfahren nach einem der vorhergehenden Ansprüche, bei dem man einen Gehalt an öliger Substanz von 0,2 bis 20 und insbesondere 1 bis 7,5 Gew.-% vorsieht (auf Basis Granulat-Gesamtgewicht).

12. Verfahren nach einem der vorhergehenden Ansprüche, bei dem man das erhaltene Granulat zusätzlich mit einer Aussenphase aus einem oder mehreren Retardierungsmitteln vorsieht.

13. Verfahren nach einem der vorhergehenden Ansprüche, bei dem man mit einem Wirbelschichtgranulator oder einem Pflugscharmischer granuliert.

14. Verfahren nach einem der vorhergehenden Ansprüche, bei dem man mit Hilfe eines Granulatbindemittels granuliert, insbesondere in Form einer Lösung (Granulierlösung) des Granulatbindemittels in einem Lösungsmittel.

15. Verfahren nach einem der vorhergehenden Ansprüche, bei dem man das erhaltene Granulat zu Tabletten Weiterverarbeitet.

16. Verfahren zur Herstellung von Tabletten, bei dem man ein Granulat zu Tabletten verarbeitet, das gemäß einem der Ansprüche 1 bis 14 erhalten worden ist.

17. Verfahren nach Anspruch 15 oder 16, bei dem man für die Weiterverarbeitung zu Tabletten oder zur Herstellung von Tabletten Hilfsstoffe verwendet, insbesondere Füllstoffe, Schmiermittel, Fließregulierungsmittel und/oder Sprengmittel.

18. Verfahren nach Anspruch 17, bei dem man die Tablette mit einem Überzug versieht.

## Claims

1. Process for the preparation of granules for a pharmaceutical formulation, wherein
(i) a mixture comprising or consisting of
- one or more active ingredients, and
- one or more retarding agents
is wetted by spraying with an oily substance selected from neutral oil, sesame oil, peanut oil, olive oil, almond oil, soybean oil, coconut oil, cottonseed oil, corn oil, rape oil, sunflower oil, wheat kernel oil, liquid paraffin, wax solutions in organic oil, and low-viscosity wax, and
(ii) the mixture is granulated.

2. Process for the preparation of granules for a pharmaceutical formulation, wherein
(i) one or more active ingredients is/are mixed with one or more retarding agents,
(ii) the mixture obtained is wetted by spraying with an oily substance selected from neutral oil, sesame oil, peanut oil, olive oil, almond oil, soybean oil, coconut oil, cottonseed oil, corn oil, rape oil, sunflower oil, wheat kernel oil, liquid paraffin, wax solutions in organic oil, and low-viscosity wax, and
(iii) the mixture obtained is granulated.

3. Process according to claim 1 or 2, wherein there is used a mixture according to claim 1 (i) or claim 2 (ii) comprising one or more excipients, especially comprising one or more fillers, flow-regulating agents, wetting agents and/or disintegrants.

4. Process according to any one of the preceding claims, wherein wetting with the oily substance is carried out at room temperature.

5. Process according to any one of the preceding claims, wherein there is provided for the mixture according to claim 1 (i) or claim 2 (ii) at least one corrosive and/or hydrophilic active ingredient.

6. Process according to any one of the preceding claims, wherein an active ingredient content of from 0.1 to 98% by weight and especially from 0.5 to 70% by weight is provided (based on the total weight of the granules).

7. Process according to any one of the preceding claims, wherein as retarding agent for the mixture according to claim 1 (i) or according to claim 2 (ii) there is provided a lipophilic retarding agent, especially in combination with a hydrogel matrix-forming agent and/or structural matrix-forming agent.

8. Process according to claim 7, wherein as retarding agent there is provided a combination of lipophilic retarding agent and hydrogel matrix-forming agent.

9. Process according to any one of the preceding claims, wherein as retarding agent there is provided a combination of lipophilic retarding agent and structural matrix-forming agent with water-soluble excipient.

10. Process according to any one of the preceding claims, wherein as oily substance there is used a natural oil, a synthetic oil, a solution of wax in oil, or low-viscosity wax.

11. Process according to any one of the preceding claims, wherein a content of oily substance is from 0.2 to 20% by weight and especially from 1 to 7.5% by weight is provided (based on the total weight of the granules).

12. Process according to any one of the preceding claims, wherein the granules obtained are in addition provided with an outer phase of one or more retarding agents.

13. Process according to any one of the preceding claims, wherein granulating is carried out using a fluidised bed granulator or a plowshare mixer.

14. Process according to any one of the preceding claims, wherein granulating is carried out with the aid of a granule binder, especially in form of a solution (granulation solution) of the granule binder in a solvent.

15. Process according to any one of the preceding claims, wherein the granules obtained are further processed to form tablets.

16. Process for the preparation of tablets, wherein granules that have been obtained according to any one of claims 1 to 14 are processed to form tablets.

17. Process according to claim 15 or 16, wherein the further processing to form tablets or for the preparation of tablets, excipients are used, especially fillers, lubricants, flow-regulating agents and/or disintegrants.

18. Process according to claim 17, wherein the tablet is provided with a coating.

## Revendications

1. Procédé de fabrication de granulés destinés à une formulation pharmaceutique, dans lequel on
(i) humidifie par pulvérisation un mélange comportant ou composé de
- un ou plusieurs principes actifs, et
- un ou plusieurs agents retardateurs, avec une substance huileuse sélectionnée parmi une huile neutre, une huile de sésame, une huile d'arachide, une huile d'olive, une huile d'amande, une huile de soja, une huile de coprah, une huile de semence de coton, une huile de maïs, une huile de colza, une huile de tournesol, une huile de germe de blé, de la paraffine liquide, des solutions cireuses dans des huiles organiques et des cires peu visqueuses, et
(ii) on granule le mélange.

2. Procédé de fabrication de granulés destinés à une formulation pharmaceutique dans lequel on
(i) mélange un ou plusieurs principes actifs avec un ou plusieurs agents retardateurs,
(ii) on humidifie par pulvérisation le mélange obtenu avec une substance huileuse sélectionnée parmi une huile neutre, une huile de sésame, une huile d'arachide, une huile d'olive, une huile d'amande, une huile de soja, une huile de coprah, une huile de semence de coton, une huile de maïs, une huile de colza, une huile de tournesol, une huile de germe de blé, de la paraffine liquide, des solutions cireuses dans des huiles organiques et des cires peu visqueuses, et
(iii) on granule le mélange obtenu.

3. Procédé selon la revendication 1 ou 2, dans lequel on utilise un mélange selon la revendication 1 (i) ou la revendication 2 (ii) présentant une teneur en un ou plusieurs adjuvants, en particulier présentant une teneur en un ou plusieurs agents de charge, agents régulateurs d'écoulement, agents humectants et/ou agents délitants.

4. Procédé selon l'une des revendications précédentes, dans lequel on humidifie avec la substance huileuse à la température ambiante.

5. Procédé selon l'une des revendications précédentes, dans lequel on prévoit pour le mélange selon la revendication 1 (i) ou la revendication 2 (ii) au moins un principe actif corrosif et/ou hydrophile.

6. Procédé selon l'une des revendications précédentes, dans lequel on prévoit une teneur en principe actif comprise de 0,1 à 98, et en particulier de 0,5 à 70 % en poids (sur la base du poids total des granulés).

7. Procédé selon l'une des revendications précédentes, dans lequel on prévoit en tant qu'agent retardateur pour le mélange selon la revendication 1 (i) ou la revendication 2 (ii) un agent retardateur lipophile, en particulier en combinaison avec un agent formateur de matrice d'hydrogel et/ou de matrice de structure.

8. Procédé selon la revendication 7, dans lequel on prévoit en tant qu'agent retardateur une combinaison d'agent retardateur lipophile et d'agent formateur de matrice d'hydrogel.

9. Procédé selon l'une des revendications précédentes, dans lequel on prévoit en tant qu'agent retardateur une combinaison d'agent retardateur lipophile et d'agent formateur de matrice de structure avec un adjuvant hydrosoluble.

10. Procédé selon l'une des revendications précédentes, dans lequel on utilise en tant que substance huileuse une huile naturelle, une huile de synthèse, une solution de cire dans de l'huile ou une cire peu visqueuse.

11. Procédé selon l'une des revendications précédentes, dans lequel on prévoit une teneur en substance huileuse comprise de 0,2 à 20 et en particulier de 1 à 7,5 % en poids (sur la base du poids total des granulés).

12. Procédé selon l'une des revendications précédentes, dans lequel on prévoit en plus des granulés obtenus une phase externe composée d'un ou de plusieurs agents retardateurs.

13. Procédé selon l'une des revendications précédentes, dans lequel on effectue la granulation avec un granulateur en lit fluidisé ou un mélangeur à soc.

14. Procédé selon l'une des revendications précédentes, dans lequel on granule à l'aide d'un liant pour granulés, en particulier sous la forme d'une solution (solution de granulation) du liant pour granulés dans un solvant.

15. Procédé selon l'une des revendications précédentes, dans lequel on transforme les granulés obtenus en comprimés.

16. Procédé de fabrication de comprimés dans lequel on transforme en comprimés des granulés qui ont été obtenus selon l'une des revendications 1 à 14.

17. Procédé selon la revendication 15 ou 16, dans lequel on utilise pour la transformation en comprimés ou pour la fabrication de comprimés des adjuvants, en particulier des matières de charge, des lubrifiants, des agents régulateurs d'écoulement et/ou des agents délitants.

18. Procédé selon la revendication 17, dans lequel on munit les comprimés d'un enrobage.
